Europäisches Patentamt

European Patent Office

Office européen des brevets

(19)

(11) **EP 1 323 378 A2**

(12) **EUROPÄISCHE PATENTANMELDUNG**

(43) Veröffentlichungstag:
**02.07.2003  Patentblatt 2003/27**

(51) Int Cl.⁷: **A61B 6/03**

(21) Anmeldenummer: **02102692.7**

(22) Anmeldetag: **06.12.2002**

(84) Benannte Vertragsstaaten:
**AT BE BG CH CY CZ DE DK EE ES FI FR GB GR
IE IT LI LU MC NL PT SE SI SK TR**
Benannte Erstreckungsstaaten:
**AL LT LV MK RO**

(30) Priorität: **07.12.2001  DE 10160205**

(71) Anmelder:
• **Philips Intellectual Property & Standards GmbH
20099 Hamburg (DE)**
• **Koninklijke Philips Electronics N.V.
5621 BA Eindhoven (NL)**

(72) Erfinder:
• **Koehler, Thomas
52088, Aachen (DE)**
• **Grass, Michael
52088, Aachen (DE)**
• **Rasche, Volker
52066, Aachen (DE)**

(74) Vertreter: **Volmer, Georg, Dipl.-Ing. et al
Philips Intellectual Property & Standards GmbH,
Postfach 50 04 42
52088 Aachen (DE)**

(54) **Verfahren zur Rekonstruktion eines 3D-Bilddatensatzes eines Untersuchungsbereiches**

(57)  Verfahren zur Rekonstruktion eines 3D-Bilddatensatzes eines Untersuchungsbereichs (13), in dem ein sich näherungsweise periodisch bewegendes Untersuchungsobjekt, z.B. das Herz, angeordnet ist. Segmente (40,50,60) des Untersuchungsbereiches (13) werden aus einem von der Detektoreinheit (16) erfassten Messdatensatz rekonstruiert, der stückweise während einer Anzahl n von periodisch aufeinanderfolgenden Zeitintervallen $\Delta t$ erfasst wird, wobei die Zeitintervalle $\Delta t$ kleiner als die Periode T sind und mit der Periode T aufeinanderfolgen. Die Rotation der Strahlenquelle (S) um die Rotationsachse (14) wird derart gesteuert wird, dass die Strahlenquelle (S) während der n Zeitintervalle $\Delta t$ insgesamt um einen Winkelbereich um die Rotationsachse (14) rotiert wird, der größer oder gleich einer Summe von 180° und einem Winkel $\beta$ ist, wobei der Winkel $\beta$ einen Öffnungswinkel des kegelförmigen Strahlenbündels (4) in einer Ebene senkrecht zu der Rotationsachse (14) darstellt. Die Translation der Strahlenquelle (S) relativ zu dem Untersuchungsbereich (13) in Richtung der Rotationsachse (14) wird derart gesteuert wird, dass das kegelförmige Strahlenbündel (4) das Segment (40,50,60) des Untersuchungsbereichs (13) jederzeit während der n Zeitintervalle $\Delta t$ vollständig durchstrahlt.

Fig. 1

EP 1 323 378 A2

**Beschreibung**

Die vorliegende Erfindung betrifft ein Verfahren zur Rekonstruktion eines 3D-Bilddatensatzes eines Untersuchungsbereichs, in dem ein sich näherungsweise periodisch bewegendes Untersuchungsobjekt angeordnet ist, mit den Schritten:

- Erfassen einer Periode T der Bewegung des Untersuchungsobjektes,
- Erfassen eines von einer Strahlenquelle emittierten, kegelförmigen Strahlenbündels nach Durchtreten des zwischen der Strahlenquelle und einer Detektoreinheit einer Abtasteinheit angeordneten Untersuchungsbereiches,
- Erzeugen einer um eine Rotationsachse verlaufenden, helixförmigen Relativbewegung zwischen der Abtasteinheit und dem Untersuchungsobjekt mittels einer Antriebseinheit,
- Rekonstruktion eines 3D-Bilddatensatzes eines Segments des Untersuchungsbereiches aus einem von der Detektoreinheit erfassten Messdatensatz, der stückweise während einer Anzahl n von periodisch aufeinanderfolgenden Zeitintervallen Δt erfasst wird, wobei die Zeitintervalle Δt kleiner als die Periode T sind und mit der Periode T aufeinanderfolgen.

Ein solches Verfahren ist aus der EP 0 983 747 A1 bekannt. Bei diesem Verfahren wird das Herz eines Patienten untersucht. Ein Elektrokardiograph wird dazu eingesetzt, die periodischen Bewegungen des Herzens zu erfassen. Die Daten des Elektrokadigraphen werden dazu verwendet, die Phasen der Herzbewegung des Patienten mit den von der Detektoreinheit erfassten Daten zu korrelieren. Eine Bildrekonstruktionseinheit rekonstruiert Bilder des Patienten auf der Grundlage der von dem Elektrokardiografen und der Detektoreinheit erfassten Daten.

Der Einsatz der Computertomographie zur Bildgebung im Herzbereich führt häufig zu Bildern mit Artefakten aufgrund der Herzbewegung während der Datenerfassung. Um diese Artefakte zu reduzieren, werden nur diejenigen Messdaten ausgewertet, die während Herzbewegungsphasen mit geringer Bewegung erfasst wurden. Die Auswahl der auszuwertenden Messdaten erfolgt anhand des zeitgleich aufgenommenen Elektrokardiogrammes. Allerdings muss dabei sichergestellt werden, dass zur Rekonstruktion ausreichende Daten aus solchen Herzbewegungsphasen mit geringer Bewegung des Herzens vorliegen, um überhaupt eine Rekonstruktion des 3D-Bilddatensatzes durchführen zu können.

Der Erfindung liegt die Aufgabe zugrunde, ein Verfahren der eingangs genannten Art weiter zu verbessern und insbesondere eine möglichst exakte Rekonstruktion des Untersuchungsobjektes zu ermöglichen.

Diese Aufgabe wird erfindungsgemäß gelöst, indem die Rotation der Strahlenquelle um die Rotationsachsen derart gesteuert wird, dass die Strahlenquelle während der n Zeitintervalle Δt insgesamt um einen Winkelbereich um die Rotationsachse rotiert wird, der größer oder gleich einer Summe von 180° und einem Winkel von β ist, wobei der Winkel β einen Öffnungswinkel des kegelförmigen Strahlenbündels in einer Ebene senkrecht zu der Rotationsachse darstellt, und die Translation der Strahlenquelle relativ zu dem Untersuchungsbereich in Richtung der Rotationsachse derart gesteuert wird, dass das kegelförmige Strahlenbündel das Segment des Untersuchungsbereiches jederzeit während der n Zeitintervalle Δt vollständig durchstrahlt.

Die Erfassung der Periodizität der Bewegung des Untersuchungsobjektes dient also nicht nur dazu, eine zeitliche Korrelation zwischen den erfassten Messdaten und der Phase der Bewegung des Untersuchungsobjektes herzustellen, sondern auch dazu, die Rotation und Translation der Strahlenquelle relativ zu dem Untersuchungsbereich zu steuern. Die während der Zeitintervalle Δt aufgenommenen Messdaten sind jeweils mit einer Phase der Bewegung des Untersuchungsobjektes korreliert.

Zur Rekonstruktion einer Schicht des Untersuchungsbereiches ist es notwendig, jeden Punkt des Untersuchungsbereiches aus einem Winkelbereich von 180° zu durchstrahlen. Für Punkte des Untersuchungsbereiches, die auf der Rotationsachse der Strahlenquelle liegen, ist diese Bedingung erfüllt, wenn die Strahlenquelle insgesamt um 180° um die Rotationsachse rotiert wird. Für Punkte am Rande des Untersuchungsbereiches muss jedoch eine Rotation durchgeführt werden, die größer als 180° ist. Die Größe des rekonstruietbaren Untersuchungsbereiches wird durch den Öffnungswinkel β in der zur Rotationsachse senkrechten Ebene begrenzt. Nur Punkte, die jederzeit innerhalb des Strahlkegels liegen, können rekonstruiert werden. Der gesamte Winkel von 180°+β ist gerade so gewählt, dass jeder Punkt des Untersuchungsbereiches aus allen Richtungen innerhalb eines Winkel von 180° bei der Aufnahme durchstrahlt wird. Die Durchstrahlung der Punkte aus allen diesen Richtungen erfolgt nicht kontinuierlich, sondern stückweise während n aufeinanderfolgender Zeitintervalle Δt.

Die vorstehende Bedingung, nämlich dass jeder Punkt des Untersuchungsbereichs während der n Zeitintervalle Δt durchstrahlt wird bedingt, dass die Translation der Strahlenquelle relativ zu dem Untersuchungsbereich geeignet gesteuert wird. Die Strahlenquelle bewegt sich kontinuierlich in Richtung der Rotationsachse relativ zu dem Untersuchungsbereich, was dazu führt, dass das kegelförmige Strahlenmündel nach einer gewissen Zeit ein vollständig anderes Segment des Untersuchungsbereichs durchstrahlt als zuvor. Der während der n Zeitintervalle Δt erfasste Messdatensatz ist jedoch nur dann zur Rekonstruktion eines Bildes des Untersuchungsbereiches geeignet, wenn die erfassten Messdaten jeweils aus demselben Segment des Untersuchungsbereiches stammen. Die Translationsbewegung der Strahlenquelle muss deshalb derart gesteuert werden, dass das kegelförmige Strahlenbündel der Strahlenquelle das Segment des Untersuchungsbereiches jederzeit während der n Zeitintervalle $\Delta t$ vollständig durchstrahlt.

Bevorzugt wird die Strahlenquelle während eines Zeitintervalls $\Delta t$ um einen Winkelbereich $\Delta \lambda$ um die Rotationsachse rotiert, der größer oder gleich

$(180°+\beta)/n$ ist. Wenn die während n Zeitintervallen $\Delta t$ überstrichenen Winkelbereiche $\Delta \lambda$ aneinander anschließen, so entspricht der gesamte überstrichene Winkelbereich $n*\Delta\lambda=180°+\beta$. Der Winkelbereich $\Delta \lambda$ wird bevorzugt etwas größer als $(180°+\beta)/n$ gewählt, so dass die aufeinanderfolgenden Winkelbereiche $\Delta \lambda$ teilweise überlappen.

Die Strahlenquelle kann während der Periode T um einen Winkel $\varphi$ um die Rotationsachse rotiert werden, der entweder im wesentlichen $360°+\Delta\lambda$ oder $360°-\Delta\lambda$ beträgt. Während n periodisch aufeinanderfolgender Zeitintervalle $\Delta t$ muss die Strahlenquelle um einen Gesamtwinkel von $180°+\beta$ um den Untersuchungsbereich rotiert werden. Wenn die Strahlenquellen während der Periode T um $360°$ rotiert würde, so würde während jedes Zeitintervalls $\Delta t$ derselbe Winkelbereich überstrichen. Die Rotationsgeschwindigkeit der Strahlenquelle muss demnach so gewählt werden, dass die während der Zeitintervalle $\Delta t$ aufgenommenen n Winkelbereiche $\Delta \lambda$ aneinander anschließen. Dafür ist gesorgt, wenn die Rotationsgeschwindigkeit wie vorstehend dargelegt definiert wird.

Der gesamte Untersuchungsbereich kann rekonstruiert werden, indem 3D-Bilddatensätze mehrerer aufeinanderfolgender Segmente des Untersuchungsbereiches aus von der Detektoreinheit erfassten Messdatensätzen rekonstruiert werden, wobei jeder Messdatensatz während einer Anzahl n von messdatensatzspezifischen periodisch aufeinanderfolgenden Zeitintervallen $\Delta t$ erfasst wird. Jedes Segment des Untersuchungsbereiches wird demnach mittels des erfindungsgemäßen Verfahrens rekonstruiert, und der gesamte Untersuchungsbereich ergibt sich, indem die Segmente aneinandergefügt werden. Die Zeitintervalle $\Delta t$ unterschiedlicher Messdatensätze können jedoch teilweise identisch sein So können beispielsweise die letzten n-1 Zeitintervalle für ein erstes Segment mit den ersten n-1 Zeitintervallen für ein zweites Segment übereinstimmen. Die messdatensatzspezifischen Zeitintavalle $\Delta t$ müssen lediglich so gewählt werden, dass sie die Rekonstruktion aufeinanderfolgender Segmente des Untersuchungsbereichs erlauben.

Die Translation in Richtung der Rotationsachse wird bevorzugt derart gesteuert, dass jedes der Segmente des Untersuchungsbereichs während der entsprechenden messdatensatzspezifischen Zeitintervalle $\Delta t$ von einem kegelförmigen Strahlenbündel vollständig durchstrahlt wird. Damit wird gewährleistet, dass jedem der Segmente ein Datensatz zugeordnet werden kann, der zur Rekonstruktion eines Bildes des Segmentes ausreicht.

Die Translation der Strahlenquelle wird bevorzugt derart gesteuert, dass die Translation P nach einer Rotation der Strahlenquelle um $360°$ um die Rotationsachse kleiner oder gleich

$$\frac{Hd}{nD\left(1-\dfrac{(n-1)\Delta\lambda}{n*360°}\right)}$$

ist, wenn der Winkel $\varphi$ im wesentlichen $360°-\Delta\lambda$ entspricht, wobei D einem Abstand der Detektoreinheit von der Strahlenquelle, H einer Höhe des Strahlenbündels in Richtung der Rotationsachse im Abstand D zu der Strahlenquelle und d einem konstanten Abstand des Untersuchungsbereichs von der Strahlenquelle entspricht.

Die Translation P entspricht dem Abstand benachbarter Windungen der Helix in Richtung der Rotationsachse zueinander. Dieser Abstand muss so gewählt werden, dass nach Ablauf von n Zeitintervallen $\Delta t$ die Strahlenquelle dasselbe zu rekonstruierende Segment des Untersuchungabereichs durchstrahlt. Dies hängt insbesondere von dem Öffnungswinkel des kegelförmigen Strahlenbündels in Richtung der Rotationsachse ab. Je größer dieser Winkel ist, desto größer kann auch eine Translation P gewählt werden, die gewährleistet, dass derselbe Untersuchungsbereich durchstrahlt wird. Die Höhe H des Strahlenbündels in Richtung der Rotationsachse im Abstand D zu der Strahlenquelle geteilt durch den Abstand D ist ein Maß für den Öffnungswinkel in Richtung der Rotationsachse.

Ferner sollen die rekonstruierbaren Segmente des Untersuchungsbereiches lückenlos aufeinander folgen. Die n Zeitintervalle $\Delta t$, die einem Messdatensatz zugeordnet sind, sollen sich jeweils aus dem letzten n-1 Zeitintervall des vorherigen Messdatensatzes und dem ersten n-1 Zeitintervall des nachfolgenden Messdatensatzes zusammensetzen. Wenn das rekonstruierbare Segment des Untersuchungsbereiches eine Höhe h in Richtung der Rotationsachse besitzt, so kann mittels der vorstehend beschriebenen Messdatensätze ein aus den Segmenten zusammengesetzter und zusammenhängender Bereich des Untersuchungsbereichs rekonstruiert werden, wenn die den Messdatensätzen jeweils zuzuordnenden Zeitintervalle $\Delta t$ bzw. Winkelbereiche $\Delta \lambda$ um die Höhe h jedes Segments zueinander räumlich verschoben sind Dieser Abstand wird letztlich durch die Translationsgeschwindigkeit festgelegt, wofür die Translation P nach einer Rotation der Strahlenquelle um 360° um die Rotationsachse ein Maß ist. Wenn die Translation P gemäß der vorstehenden Formel gewählt ist, dann lässt sich der gesamte Untersuchungsbereich anhand der aufeinanderfolgenden Segmente wie vorstehend beschrieben rekonstruieren. Wenn die Strahlenquelle während der Periode T um 360°+Δλ um die Rotationsachse rotiert wird, so ist für die Translation P ein Wert kleiner oder gleich

$$\frac{Hd}{nD\left(1+\dfrac{(n+1)\Delta\lambda}{n*360°}\right)}$$

aus denselben Gründen zu wählen.

Zur Bestimmung der Periode T kann die Bewegung des Untersuchungsobjektes mehrfach gemessen werden und bei unterschiedlichen Messwerten für die Periode T ein größter Messwert für die Periode T gewählt werden. Diese Vorgehensweise bietet sich an, wenn sich dass zu untersuchende Objekt nicht exakt periodisch verhält. Die Wahl eines möglichst großen Messwertes hat zur Folge, dass die tatsächliche Rate während der Abtastung der Messwerte im Mittel kleiner als die gewählte Periode ist.

Bevorzugt wird die Erfindung zur Rekonstruktion des Herzens angewendet. Die Periode des Herzschlages wird dann vorzugsweise mittels eines Elektrokardiografen erfasst.

Eine Vorrichtung zur Durchführung des erfindungsgemäßen Verfahrens ist in Anspruch 10 definiert. Die Vorrichtung umfasst eine Erfassungseinheit, eine Abtasteinheit, eine Antriebseinheit, eine Steuereinheit und eine Rekonstruktionseinheit, die zur Durchführung des Verfahrens geeignet ausgebildet sind

Die Erfindung wird nachfolgend anhand der Zeichnungen näher erläutert.

Es zeigen:

| | |
|---|---|
| Figur 1 | eine erfindungsgemäße Vorrichtung in schematischer Darstellung, |
| Figuren 2a, 2b und 2c | drei seitliche Ansichten einer helixförmigen Abtastbahn, |
| Figur 3 | eine Draufsicht auf die helixförmige Abtastbahn der Röntgenquelle und den Untersuchungsbereich, |
| Figuren 4a, 4b, 4c | jeweils weitere seitliche Ansichten der helixförmigen Abtastbahn, |
| Figur 5 | eine seitliche Schnittansicht des kegelförmigen Strahlenbündels einer Röntgenquelle, und |
| Figur 6 | mehrere aufeinanderfolgende zylinderförmige Segmente des Untersuchungsbereichs. |

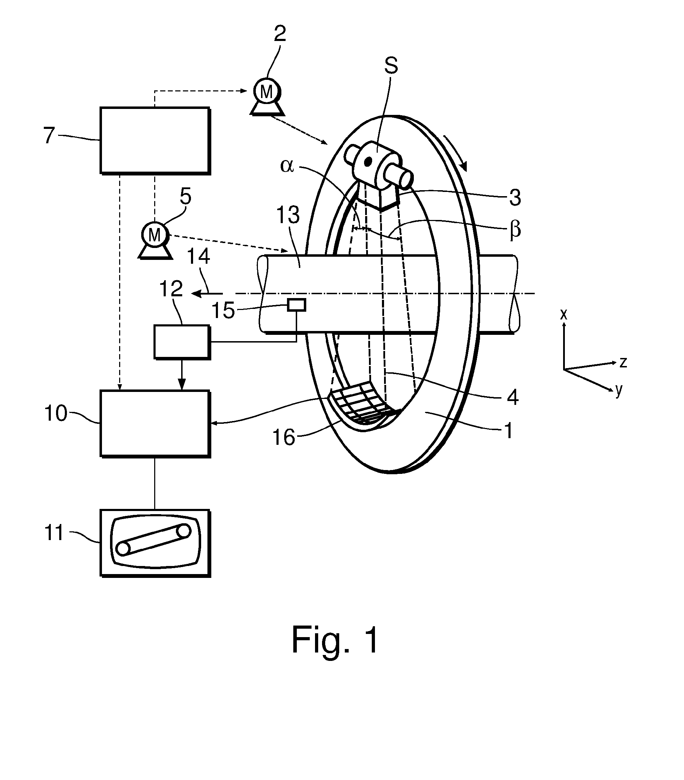

Der in Figur 1 dargestellte Computertomograph umfasst eine Gantry 1, die um eine parallel zur z-Achse verlaufende Rotationsachse 14 rotieren kann. Dazu wird die Gantry 1 von einem Motor 2 mit einer vorzugsweise konstanten aber steuerbaren Winkelgeschwindigkeit angetrieben. An der Gantry 1 ist eine Strahlenquelle S, beispielsweise eine Röntgenröhre, befestigt. Diese ist mit einer Kollimatoranordnung 3 versehen, die aus der von der Strahlenquelle S erzeugten Strahlung ein kegelförmiges Strahlenbündel 4 ausblendet. Das Strahlenbündel 4 durchdringt ein nicht dargestelltes Untersuchungsobjekt, das sich in einem zylinderförmigen Untersuchungsbereich 13 befindet. Nach dem Durchsetzen des Untersuchungsbereichs 13 trifft das Strahlenbündel 4 auf eine an der Gantry 1 befestigte zweidimensionale Detektoreinheit 16. Den mit β bezeichneten Öffnungswinkel des Strahlenbündels 4 (als Öffnungswinkel ist der Winkel definiert, den die in der xy-Ebene am Rande liegenden Strahlen des Bündels 4 einschließen) bestimmt den Durchmesser des Untersuchungsbereichs 13, innerhalb dessen das zu untersuchende Objekt sich bei der Akquisition der Messwerte befinden muss Der dabei beispielsweise auf einem Patientenlagerungstisch liegende, im Untersuchungsbereich 13 angeordnete Patient kann mittels eines Motors 5 parallel zur Richtung der Rotationsachse 14 bzw. der z-Achse verschoben werden.

Der mit α bezeichnete Öffnungswinkel des Strahlenbündels 4 ist durch den Winkel definiert, den am Rande liegende Strahlen des Strahlenbündels 4 einschließen, die in der durch die Rotationsachse 14 und die Strahlenquelle S definierten Ebene liegen. Der Öffnungswinkel α bestimmt das Segment des Untersuchungsbereichs, welches während einer Rotation um die Rotationsachse 14 durchstrahlt wird.

Die von der Detektoreinheit 16 akquirierten Messdaten werden einer Rekonstruktionseinheit 10 zugeführt, die daraus die Absorptionsverteilung in dem von dem Strahlenkegel 4 erfassten Teil des Untersuchungsbereichs 13 rekonstruiert und beispielsweise auf einem Monitor 11 wiedergibt. Die beiden Motoren 2 und 5, die Rekonstruktionseinheit 10, die Strahlenquelle S und der Transfer der Messdaten von der Detektoreinheit 16 zur Rekonstruktionseinheit 10 werden von einer geeigneten Steuereinheit 7 gesteuert.

Die Steuerung der Motoren 2 und 5 erfolgt derart, dass das Verhältnis der Vorschubgeschwindigkeit des Untersuchungsbereichs 13 und die Winkelgeschwindigkeit der Gantry 1 in einem konstanten Verhältnis stehen, so dass sich Strahlenquelle S und Untersuchungsbereich 13 relativ zueinander auf einer helixförmigen Bahn, der sogenannten Trajektorie, bewegen. Dabei ist es gleichgültig, ob die Abtasteinheit aus Strahlenquelle S und Detektor 16 oder der Untersuchungsbereich 13 die Rotations- bzw. Vorschubbewegung ausführen; wesentlich ist allein die Relativbewegung.

Bevorzugt wird gleichzeitig mit der Erfassung der Messdaten ein Herzbewegungssignal mittels eines Elektrokardiografen 12 und eines an dem Patienten angebrachten Sensors 15 erfasst. Aus dem von dem Elektrokardiografen aufgenommenen Elektrokardiogramm kann die Periode T des Herzschlages ermittelt werden. Das Elektrokardiogramm wird ebenfalls der Rekonstruktionseinheit zugeführt, um dadurch die Auswahl der für die Rekonstruktion geeigneten Messdaten vorzunehmen. Vorzugsweise werden nur aus bewegungsarmen Herzbewegungsphasen erfasste Messdaten ausgewertet. Die Steuereinheit 12 steuert anhand des Herzbewegungssignals die Rotations- und Translationsbewegung der Röntgenquelle relativ zu dem Untersuchungsbereich 13, so dass die während der bewegungsarmen Herzbewegungsphasen erfassten Messdaten eine Rekonstruktion des Untersuchungsbereichs 13 erlauben.

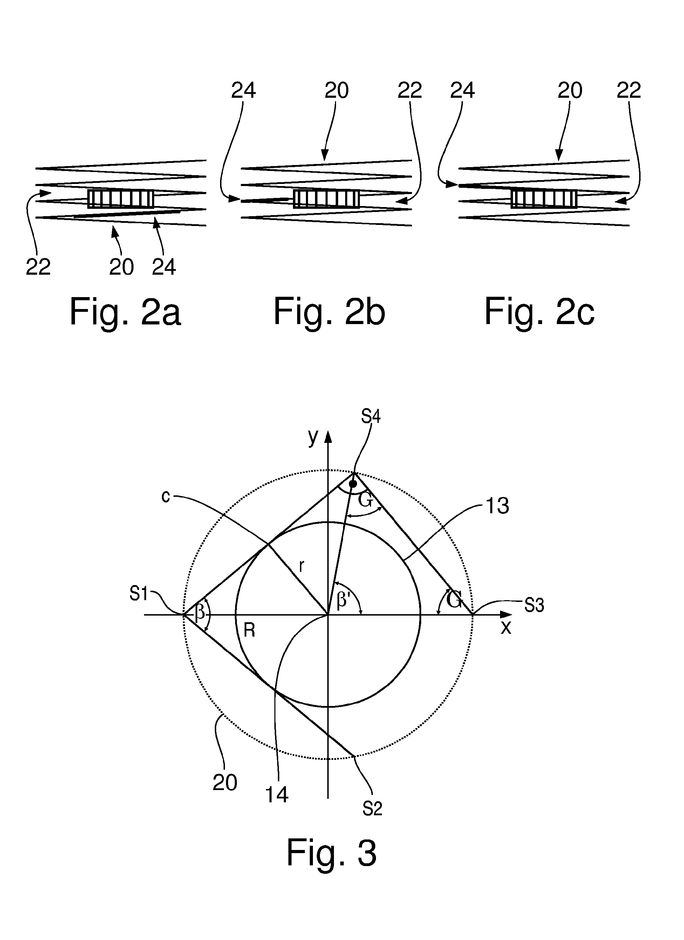

Die Figuren 2a, 2b und 2c zeigen nebeneinander drei Ansichten der Trajektorien 20 der Strahlenquelle S und jeweils ein Segment 22 des Untersuchungsbereiches 13. Die Abschnitte 24 der Trajektorie 20 stellen jeweils diejenigen Abschnitte dar, die von der Strahlenquelle S während mit der Periode T aufeinanderfolgender Zeitabschnitte $\Delta t$ überstrichen werden. Das Segment 22 des Untersuchungsbereichs 13 wird während jedes der dargestellten Abschnitte 24 von dem kegelförmigen Strahlenbündel vollständig durchstrahlt, das von der Strahlenquelle S ausgeht. Während die Strahlenquelle S entlang der drei dargestellten Abschnitte 22 der Trajektorie verfahren wird, wird der Untersuchungsbereich insgesamt um einen Winkel von $180°+\beta$ umfahren.

Figur 3 zeigt eine Draufsicht auf die Trajektorie 20 und den zylinderförmigen Untersuchungsbereich 13. Der Radius r des Untersuchungsbereichs 13 wird durch den Öffnungswinkel $\beta$ des von der Strahlenquelle S emittierten kegelförmigen Strahlenbündels und dem Abstand R der Strahlenquelle von der Rotationsachse bestimmt. Die Punkte s1, das Zentrum des Koordinatensystems, welches auf der Rotationsachse 14 liegt, und der Punkt c, an dem der Strahl s1-s4 den Untersuchungsbereich 13 berührt, bilden ein rechtwinkliges Dreieck. Für den Winkel $\beta$ gilt somit: $\sin(\beta/2) = r/R$.

Damit der dargestellte Querschnitt des Untersuchungsbereiches 13 rekonstruiert werden kann, muss jeder Punkt innerhalb des Untersuchungsbereiches aus einem Winkelbereich von 180° durchstrahlt werden. Dies gilt insbesondere für den dargestellten Punkt c, welcher auf der Peripherie des Untersuchungsbereichs 13 liegt. Dazu ist es erforderlich, dass die Strahlenquelle S von dem Punkt s1 über die Punkte s2, s3 bis zu dem Punkt s4 entlang der Trajektorie 20 verfahren wird. Dabei wird die Strahlenquelle S um einen Winkel von 180° plus dem Winkel $\beta'$ um die Rotationsachse 14 rotiert. Um zu verstehen, weshalb der von den Geraden s4-14 und s3-14 aufgespannte Winkel dem Öffnungswinkel $\beta$ des kegelförmigen Strahlenbündels entspricht ist eine Hilfsgrade s3-s4 eingezeichnet. Das durch die Punkte Zentrum des Koordinatensystems, s3 und s4 definierte Dreieck ist ein gleichschenkliges Dreieck, da die Punkte s3 und s4 im konstanten Abstand R zum Koordinatemittelpunkt liegen. Die Winkelsumme für dieses Dreieck beträgt $\beta'+2\gamma=180°$. Das durch die Punkte s1, s3 und s4 gebildete Dreieck ist ein rechtwinkliges Dreieck (THALES-Satz). Die Winkelsumme für dieses Dreieck beträgt $\beta/2+\gamma=90°$. Daraus folgt, $\beta+2\gamma=180°$. Aus den Gleichungen $\beta'+2\gamma=180°$ und $\beta+2\gamma=180°$ folgt, dass die beiden in Fig. 2 eingezeichneten Winkel $\beta'$ und $\beta$ gleichgroß sind.

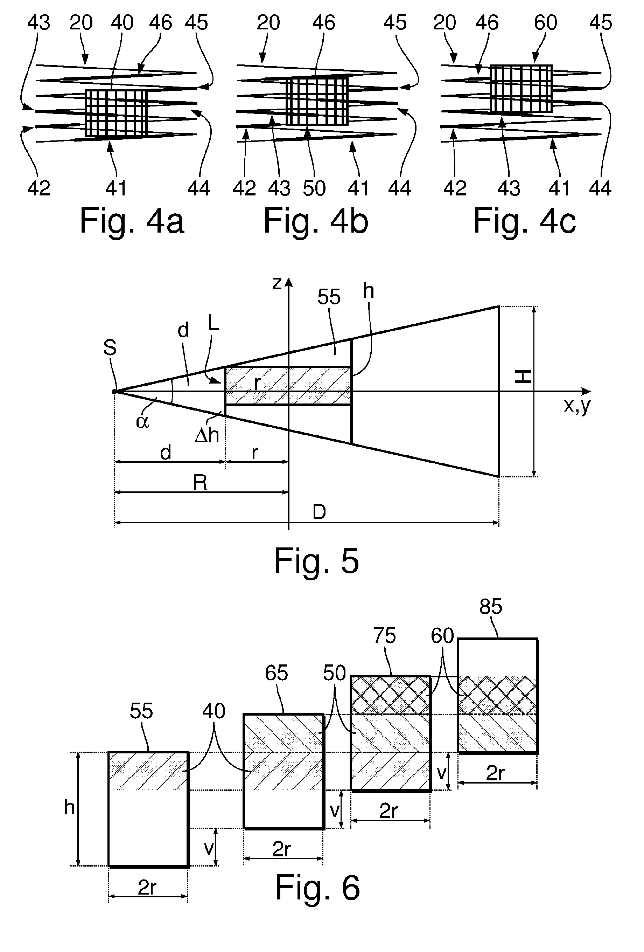

Die Figuren 4a, 4b und 4c zeigen mehrere Ansichten der Trajektorie 20 der Strahlungsquelle S nebeneinander. Die Trajektorie 20 schließt jeweils ein Segment 40, 50 und 60 des Untersuchungsbereiches ein. Die Größe der Segmente 40, 50 und 60 ist identisch, sie sind jedoch jeweils in Richtung der nicht dargestellten Rotationsachse 14 zueinander versetzt. Die Trajektorie 20 weist mehrere Abschnitte 41, 42, 43, 44, 45 und 46 auf. Die Strahlenquelle S wird kontinuierlich entlang der Trajektorie 20 mit konstanter Geschwindigkeit verfahren. Die Rotations- und Translationsgeschwindigkeit der Strahlenquelle S wird derart gesteuert, dass die Strahlenquelle S jeweils während eines der Zeitintervalle $\Delta t$ einen der Abschnitte 41 bis 46 überstreicht. Die Zeitintervalle $\Delta t$ folgen mit der Periode T der Bewegung des zu untersuchenden Objekts aufeinander. Jedes der Zeitintervalle $\Delta t$ entspricht einer identischen Phase der periodischen Bewegung des Untersuchungsobjekts.

Die Strahlenquelle S wird jeweils während eines Zeitintervalls der Längs $\Delta t$ entlang eines der Abschnitte 41 bis 46 verfahren. Dabei wird die Strahlenquelle um einen Winkel $\Delta \lambda$ um die Rotationsachse 14 rotiert. Während die Strahlenquelle S entlang der Abschnitte 42, 43 und 44 der Trajektorie verfahren wird, wird das Segment 40 des Untersuchungsbereichs vollständig durchstrahlt; während die Strahlenquelle S entlang der Abschnitte 43, 44 und 45 verfahren wird, wird das Segment 50 des Untersuchungsbereichs vollständig durchstrahlt. Das Segment 60 wird von den Abschnitten 44, 45 und 46 der Trajektorie aus vollständig durchstrahlt.

Die Höhe und Lage der Segmente 40, 50 und 60 hängt von der Lage der entsprechenden Abschnitte der Trajektorie sowie von dem Öffnungswinkel $\alpha$ der Strahlungsquelle S ab. Die aufeinanderfolgenden Abschnitte 41 bis 46 sind jeweils derart auf der Trajektorie 20 angeordnet, dass sie lückenlos aneinandergefügt werden können, indem sie entlang der Rotationsachse verschoben werden. Die Abschnitte 41 bis 46 können auch teilweise überlappen, wenn sie wie vorstehend beschrieben aneinandergefügt werden. Drei aneinanderfügte Abschnitte der Trajektorie müssen jedoch einen Winkelbereich von 180°+β um die Rotationsachse 14 einschließen. Nur dann kann das den drei Abschnitten entsprechende Segment 40, 50 oder 60 des Untersuchungsbereichs 13 rekonstruiert werden. Die Wahl der Abschnitte 41 bis 46 in Figur 4 ist beispielhaft. Ein Winkel von 180°+β kann naturgemäß auch von einer größeren oder kleineren Anzahl n von Abschnitten der Trajektorie eingeschlossen werden. Die vorliegende Wahl n=3 ist also nur beispielhaft.

Die in den Figuren 4a bis 4c dargestellten, rekonstruierbaren Segmente 40, 50 und 60 des Untersuchungsbereichs 13 überlappen. Die überlappenden Bereiche der Segmente 40, 50 und 60 können also mehrfach rekonstruiert werden. Es ist allerdings auch möglich die Trajektorie und Geschwindigkeit der Röntgenquelle S auf der Trajektorie derart zu wählen, dass die rekonstruierbaren Segmente 40, 50 und 60 lückenlos aneinander anschließen, ohne dass sie überlappen. Dies ist beispielhaft in Fig 6 gezeigt.

Figur 5 zeigt eine seitliche Schnittansicht des von der Strahlenquelle S emittierten kegelförmigen Strahlenbündels (4). Der Öffnungswinkel $\alpha$ des kegelförmigen Strahlenbündels bestimmt eine maximale Höhe 1 eines zylinderförmigen Segments des Untersuchungsbereichs, das während eines beliebigen Zeitpunktes durchstrahlt wird Während eines Zeitintervalls $\Delta t$ bewegen sich die Strahlenquellen auf der helixförmigen Bahn 20 in Richtung der z-Achse. Während dieses Zeitraumes wird die Strahlenquelle um $\Delta h$ in Richtung der z-Achse bewegt. Das am Anfang und am Ende eines Zeitintervalls $\Delta t$ durchstrahlte zylinderförmige Segment 55 des Untersuchungsbereiches 13 ist in Figur 5 schraffiert dargestellt. Die Höhe h des zylinderförmigen Segments 55 bestimmt sich durch h=1-$\Delta h$. Eine helixförmige Bahn um die z-Achsen kann durch die Gleichung $A(\lambda)=(R\cos(\lambda), R\sin(\lambda), P\lambda/2\pi)$ beschrieben werden. Wenn die Strahlenquelle S während des Zeitintervalls $\Delta t$ um einen Winkel $\Delta\lambda$ um die Rotationsachse 14 bzw. z rotiert wird, so entspricht die Höhenverschiebung $\Delta h=P\Delta\lambda/2\pi$. Wird der Winkel $\Delta\lambda$ in Grad angegeben, so gilt $\Delta h=P\Delta\lambda/360°$. Im Abstand D von der Strahlenquelle S befindet sich die Detektoreinheit 16. Das von der Strahlenquelle S emittierte Strahlenbündel 4 hat im Abstand D die Höhe H. R kennzeichnet den Abstand der Strahlenquelle S von der Rotationsachse 14 und r den Radius des Untersuchungsbereiches 13. Aufgrund der helixförmigen Bewegung der Strahlenquelle S ist der Abstand D der Strahlenquelle S von dem Untersuchungsbereich 13 während der Rotation der Strahlenquelle S um die Rotationsachse 14 konstant. Die Höhe 1 des kegelförmigen Strahlenbündels 4 im Abstand D von der Strahlenquelle S entspricht demnach 1=d/DH (Strahlensatz). Für die Höhe h des Abschnitts 40 des Untersuchungsbereiches 13 ergibt sich somit h=Hd/D-P$\Delta\lambda$/360°.

Damit aus den Messdaten, die während einer Anzahl n von Abschnitten der Trajektorie aufgenommen werden, ein 3D-Bild rekonstruiert werden kann, müssen die während der Zeitintervalle $\Delta t$ aufgenommenen Strahlen jeweils dasselbe Segment des Untersuchungsbereiches durchdringen. Dieses Segment kann dann allein rekonstruiert werden.

Figuren 6 zeigt vier Segmente 55, 65, 75 und 85 des Untersuchungsbereichs 13, die jeweils während eines von mehreren aufeinanderfolgenden Zeitintervallen $\Delta t$ von dem von der Strahlenquelle S emittierten kegelförmigen Strahlenbündel 4 durchstrahlt werden. Die Abschnitte 55, 65, 75 und 85 besitzen jeweils eine Höhe h und eine kreisförmige Grundfläche mit dem Radius r. Der Durchmesser der Grundfläche beträgt somit 2r. Der Abstand v in Richtung der Längsachse der Segmente 55, 65, 75 und 85 bzw. in Richtung der Rotationsachse 14 zwischen den Abschnitten 55, 65, 75 und 85 ist in Figur 6 so gewählt, dass er genau 1/3 der Höhe h der Segmente 55, 65, 75 und 85 entspricht. Während eines Zeitintervalles $\Delta t$ wird jeweils eines der Segmente 55, 65, 75 und 85 durchstrahlt. Die Segmente 55, 65 und 75 überlappen derart, dass sie das schraffiert dargestelltes Segment 40 einschließen. Die Segmente 65, 75 und 85 schließen das schraffierte Segment 50 ein. Das Segment 60 wird von den Segmenten 75, 85 und einem weiteren nicht dargestellten Segment eingsschlossen. Die Segmente 40, 50 und 60 des Untersuchungsbereiches 13 mit Höhe h/3 werden jeweils während drei aufeinanderfolgender Zeitinterfalle $\Delta t$ vollständig durchstrahlt. Während der drei Zeitintervalle wird die Strahlenquelle insgesamt um einen Winkel von 180°+β um die Rotationsachse 14 rotiert. Deshalb können die Segmente 40, 50 und 60 aus den Messdaten von drei aufeinanderfolgenden Zeitintervallen $\Delta t$ rekonstruiert werden.

Die rekonstruierbaren Segmente 40, 50 und 60 sind jeweils in Figur 6 schraffiert dargestellt. Die Segmente 40, 50 und 60 sind so gewählt, dass sie lückenlos aneinander anschließen. Jedes der Segmente 40, 50 und 60 wird von drei der Segmente 55, 65, 75 und 85 eingeschlossen. Selbstverständlich kann auch eine größere Anzahl n von Segmenten 55, 65, 75 und 85 ein rekonstruierbares Segment einschließen. Es muss dann gelten, dass v=h/n entspricht, wobei n die Anzahl der Zeitintervalle $\Delta t$ bzw. Abschnitte der Trajektorie angibt, die zur Rekonstruktion eines Segmentes des Untersuchungsbereiches erforderlich sind. Damit die Abschnitte der Trajektorie den Untersuchungsbereich 13 insgesamt um einen Winkel von 180°+β einschließen, muss die Röntgenquelle entweder um einen Winkel von 2π-$\Delta\lambda$ oder um einen Winkel von 2π+$\Delta\lambda$ um die Rotationsachse 13 rotiert werden, um von einem Anfang eines der Abschnitte zu dem Anfang des nachfolgenden Abschnittes zu gelangen. Dabei wird die Strahlenquelle S in Richtung der Rotationsachsen 14 um den Abstand v verschoben. Für v=(2π+/-$\Delta\lambda$)P/360°=h/n ist eine Rekonstruktion gemäß dem vorgeschlagenen Verfahren möglich. Für h gilt weiterhin h=Hd/D-P$\Delta\lambda$/360°. Daraus ergibt sich für die Translation P der Röntgenquelle in Richtung der Rotationsachse

$$\frac{Hd}{nD\left(1+\dfrac{(n+1)\Delta\lambda}{n*360°}\right)}$$

oder

$$\frac{Hd}{nD\left(1-\dfrac{(n-1)\Delta\lambda}{n*360°}\right)}.$$

Zur Rekonstruktion eines 3D-Bildes der Segmente 40, 50 und 60 des Untersuchungsbereiches aus den aufgenommenen Bilddaten wird bevorzugt ein Feldkamp-Algorithmus nach Schaller eingesetzt. Dabei kommt eine Parker-Gewichtung zur Rekonstruktion der oberen und unteren Abschnitte des Segments zum Einsatz Das Segment wird dazu zunächst in das Zentrum eines Koordinatensystems transformiert.

Im einzelnen kann folgendermaßen verfahren werden:

(a) Eine Parker-Gewichtung ("Parker-weighting") wird an den Projektionen durchgeführt, wobei der Öffnungswinkel des kegelförmigen Strahlenbündels in Richtung der Rotationsachse und unterschiedliche Positionen der Strahlenquelle relativ zu der Rotationsachse unberücksichtigt bleiben,
(b) eine Cosinus-Gewichtung wird zur Kompensation unterschiedlicher Strahlenlängen durchgeführt,
(c) eine Rampen-Filterung wird eingesetzt, und
(d) eine Rückprojektion der 3D-Bilddaten in den eigentlichen 3D-Raum wird unter Verwendung des helikalen Feldkamp-Algorithmus durchgeführt. Die echte Lage der Strahlenquelle relativ zu der Rotationsachse wird dabei berücksichtigt.

## Patentansprüche

1.   Verfahren zur Rekonstruktion eines 3D-Bilddatensatzes eines Untersuchungsbereichs (13), in dem ein sich näherungsweise periodisch bewegendes Untersuchungsobjekt angeordnet ist, mit den Schritten:

- Erfassen einer Periode T der Bewegung des Untersuchungsobjektes,
- Erfassen eines von einer Strahlenquelle (S) emittierten, kegelförmigen Strahlenbündels (4) nach Durchtreten des zwischen Strahlenquelle (S) und Detektoreinheit (16) einer Abtasteinheit (1) angeordneten Untersuchungsbereichs (13),
- Erzeugen einer um eine Rotationsachse (14) verlaufenden, helixförmigen Relativbewegung zwischen der Abtasteinheit (1) und dem Untersuchungsobjekt mittels einer Antriebseinheit (2, 5)
- Rekonstruktion eines 3D-Bilddatensatzes eines Segments (40, 50, 60) des Untersuchungsbereiches (13) aus einem von der Detektoreinheit (16) erfassten Messdatensatz, der stückweise während einer Anzahl n von periodisch aufeinanderfolgenden Zeitintervallen $\Delta t$ erfasst wird, wobei die Zeitintervalle $\Delta t$ kleiner als die Periode T sind und mit der Periode T aufeinanderfolgen,

**<u>dadurch gekennzeichnet,</u>**
**dass** die Rotation der Strahlenquelle (S) um die Rotationsachse (14) derart gesteuert wird, dass die Strahlenquelle (S) während der n Zeitintervalle $\Delta t$ insgesamt um einen Winkelbereich um die Rotationsachse (14) rotiert wird, der größer oder gleich einer Summe von 180° und einem Winkel $\beta$ ist, wobei der Winkel $\beta$ einen Öffnungswinkel des kegelförmigen Strahlenbündels (4) in einer Ebene senkrecht zu der Rotationsachse (14) darstellt, und die Translation der Strahlenquelle (S) relativ zu dem Untersuchungsbereich (13) in Richtung der Rotationsachse (14) derart gesteuert wird, dass das kegelförmige Strahlenbündel (4) das Segment (40, 50, 60) des Untersuchungsbereichs (13) jederzeit während der n Zatintervalle $\Delta t$ vollständig durchstrahlt.

2.   Verfahren nach Anspruch 1,

**dadurch gekennzeichnet,**
**dass** die Strahlenquelle (S) während eines Zeitintervalls Δt um einen Winkelbereich Δλ um die Rotationsachse (14) rotiert wird, der größer oder gleich (180°+β)/n ist.

3. Verfahren nach Anspruch 1 oder 2,
**dadurch gekennzeichnet,**
**dass** die Strahlenquelle (S) während der Periode T um einen Winkel φ um die Rotationsachse (14) rotiert wird, der im wesentlichen 360°+Δλ oder 360°-Δλ beträgt.

4. Verfahren nach Anspruch 1,
**dadurch gekennzeichnet,**
**dass** 3D-Bilddatensätze mehrerer aufeinander folgende Segmente (40, 50, 60) des Untersuchungsbereiches (13) aus von der Detektoreinheit (16) erfassten Messdatensätzen rekonstruiert werden, wobei jeder Messdatensatz während einer Anzahl n von messdatensatzspezifischen, periodisch aufeinanderfolgenden Zeitintervallen Δt erfasst wird.

5. Verfahren nach Anspruch 4,
**dadurch gekennzeichnet,**
**dass** die Translation in Richtung der Rotationsachse (14) derart gesteuert wird, dass jedes der Segmente (40, 50, 60) des Untersuchungsbereichs (13) während der entsprechenden messdatensatzspezifischen Zeitintervalle Δt von dem kegelförmige Strahlenbündel (4) vollständig durchstrahlt wird.

6. Verfahren nach Anspruch 3,
**dadurch gekennzeichnet,**
**dass** die Translation der Strahlenquelle (S) derart gesteuert wird, dass die Translation P nach einer Rotation der Strahlenquelle (S) um 360° um die Rotationsachse (14) kleiner oder gleich

$$\frac{Hd}{nD\left(1-\dfrac{(n-1)\Delta\lambda}{n*360°}\right)}$$

ist, wenn der Winkel φ im wesentlichen 360°-Δλ entspricht,
wobei D einem Abstand der Detektoreinheit (16) von der Strahlenquelle (S), H einer Höhe des Strahlenbündels (4) in Richtung der Rotationsachse (14) im Abstand D zu der Strahlenquelle (S) und d einem konstanten Abstand des Untersuchungsbereichs (13) von der Strahlenquelle (S) entspricht.

7. Verfahren nach Anspruch 3,
**dadurch gekennzeichnet,**
**dass** die Translation der Strahlenquelle (S) derart gesteuert wird, dass nach einer Rotation der Strahlenquelle (S) um 360° um die Rotationsachse (14) die Translation P kleiner oder gleich

$$\frac{Hd}{nD\left(1+\dfrac{(n+1)\Delta\lambda}{n*360°}\right)}$$

ist, wenn der Winkelbereich φ im wesentlichen 360°-Δλ
entspricht, wobei D einem Abstand der Detektoreinheit (16) von der Strahlenquelle (S) entspricht, H einer Höhe des Strahlenbündels (4) in Richtung der Rotationsachse (14) im Abstand D zu der Strahlenquelle (S) entspricht, und d einem konstanten Abstand des Untersuchungsbereichs (13) von der Strahlenquelle (S) entspricht.

8. Verfahren nach Anspruch 1,
**dadurch gekennzeichnet,**

**dass** die Periode T der Bewegung des Untersuchungsobjektes mehrfach gemessen wird und bei unterschiedlichen Messwerten für die Periode T ein größter Messwert für die Periode T gewählt wird.

9. Verfahren nach Anspruch 1,
**dadurch gekennzeichnet,**
**dass** das Untersuchungsobjekt ein Herz ist und die Periode T der Bewegung des Herzens mittels eines Elektrokardiogramms erfasst wird.

10. Vorrichtung zur Rekonstruktion eines 3D-Bilddatensatzes eines Untersuchungsbereichs (13), in dem ein sich näherungsweise periodisch bewegendes Untersuchungsobjekt angeordnet ist, mit

- einer Erfassungseinheit (12) zum Erfassen einer Periode T der Bewegung des Untersuchungsobjektes,
- einer Abtasteinheit (1) zum Erfassen eines kegelförmigen Strahlenbündels (4) nach Durchtreten des Untersuchungsbereichs (13) mit einer Detektoreinheit (16) und einer Strahlenquelle (S), wobei die Strahlenquelle (S) ausgebildet ist, ein kegelförmiges Strahlenbündel (4) zu erzeugen, und die Strahlenquelle (S), die Detektoreinheit (16) und der Untersuchungsbereich (13) derart angeordnet sind, dass das kegelförmige Strahlenbündel (4) den Untersuchungsbereich (13) durchtritt und anschließend von der Detektoreinheit (14) erfasst wird,
- einer Antriebseinheit (2, 5) zum Erzeugen einer um eine Rotationsachse (14) verlaufenden, helixförmigen Relativbewegung zwischen der Strahlenquelle (S) und dem Untersuchungsobjekt,
- einer Rekonstruktionseinheit (1) zur Rekonstruktion eines 3D-Bilddatensatzes eines Segments (40, 50, 60) des Untersuchungsbereiches (13) aus einem von der Detektoreinheit (16) erfassten Messdatensatz, der stückweise während einer Anzahl n von periodisch aufeinanderfolgender Zeitintervalle $\Delta$t erfasst wird, wobei die Zeitintervalle $\Delta$t kleiner als die Periode T sind und mit der Periode T aufeinanderfolgen, und
- einer Steuereinheit (7) zur Steuerung der Antriebseinheit (2, 5),

**dadurch gekennzeichnet,**
**dass** die Steuereinheit (7) ausgebildet ist, die Strahlenquelle (S) derart um die Rotationsachse (14) zu rotieren, dass die Strahlenquelle (S) während der n Zeitintervalle $\Delta$t insgesamt um einen Winkelbereich um die Rotationsachse (14) rotiert wird, der größer oder gleich einer Summe von 180° und einem Winkel $\beta$ ist, wobei der Winkel $\beta$ einen Öffnungswinkel des kegelförmigen Strahlenbündels (4) in einer Ebene senkrecht zu der Rotationsachse (14) darstellt, und
die Strahlenquelle (S) relativ zu dem Untersuchungsbereich (13) in Richtung der Rotationsachse (14) derart zu bewegen, dass das kegelförmige Strahlenbündel (40) das Segment (40, 50, 60) des Untersuchungsbereichs (13) jederzeit während der n Zeitintervalle $\Delta$t vollständig durchstrahlt.

Fig. 1

Fig. 2a    Fig. 2b    Fig. 2c

Fig. 3

Fig. 4a

Fig. 4b

Fig. 4c

Fig. 5

Fig. 6